# EUROPEAN PATENT SPECIFICATION

(11) **EP 1 273 239 B1**
(45) Date of publication and mention of the grant of the patent: **04.08.2004**
(21) Application number: 02425280.1
(22) Date of filing: 03.05.2002
(51) Int. Cl.: A23L 1/0562, A23L 1/305, A23J 3/34, A23J 3/06, A61K 38/01, A23L 1/302, A23L 1/303, C07K 14/46

(54) **Uses of a composition comprising partly-hydrolyzed fish gelatin**
Verwendungen einer Zusammensetzung enthaltend teilweise hydrolysiertes Fischgelatin
Utilisations d'une composition de gélatine de poisson partiellement hydrolysée

(30) Priority: 02.07.2001 IT RM20010380
(43) Date of publication of application: 08.01.2003
(60) Divisional application: 03027512.7
(73) Proprietor: BIOPROGRESS S.p.A., I-00165 Rome (IT)
(72) Inventor: Bonanomi, Michele, Roma (IT); De Gregorio, Mauro, Roma (IT)
(74) Representative: Tansini, Elio Fabrizio

(56) References cited:
- WO-A-00/10525
- WO-A-00/61117
- DATABASE WPI Section Ch, Week 200002 Derwent Publications Ltd., London, GB; Class D13, AN 2000-016462 XP002218542 & JP 11 266803 A (ISHIZUKA Y), 5 October 1999 (1999-10-05)

## Description

The present invention relates to the use of a partly-hydrolysed fish gelatin as a medical speciality. In particular, the present invention relates to use of partly-hydrolysed fish gelatin for preparation of food additives to use as supplements in the medical field, as a source of easily assimilable amino acids useful in conditions that all have in common a deficient supply, an increased consumption or a defective absorption of amino acids, both in the human and animal field. The uses object of the invention are the treatment or prevention of tooth diseases connected with both thinning of the bone tissue and the weakening of dental ligaments, of paradontitis, and the treatment of the trophism of the micro-circulation and the veins.

It is known that gelatin obtained from connectivum contains essential and nonessential amino acids in proportions corresponding to those of the bone, the articulations, the keratin and other connective tissues. Oral administration of same to men and laboratory animals, in amounts at least as high as 100 mg/kg, showed a favourable effect under various situations and conditions. By way of example, oral administration of same to an amount of 10 g/day has a well-documented effect on pain and motility in patients suffering from arthrosis, osteoporosis and other diseases of the locomotor apparatus (Adam M., Therapiewoche (1991) 41 2456-61).

In an amount of 100 mg/kg/day it improves hair trophism both in men and laboratory animals (Silvestrini B., "Method for increasing hair growth" US Patent 4,749,684 (1988); Scala J., Hollies N.R.S., Sucher K.P., Nutr. Report Inter. (1976) 13 579-592).

Oral administration of gelatin to the above mentioned dosages is not easy and it is often badly tolerated because this substance in the stomach swells giving rise to an unpleasant feeling of repletion. In addition, in some persons it may cause reaction of the allergic type.

To avoid the above mentioned drawbacks, resorting to a completely hydrolysed gelatin improving amino acid absorption was proposed in the past, so as to enable dosage halving and avoid gastric trouble (Silvestrini B., and Kirschner G., "Integratori alimentari e dietetici contenenti gelatina idrolizzata e loro uso in campo medico" Patent IT 01299131).

However, the completely hydrolysed gelatin too has some drawbacks. In patent IT 01299131 the gelatin hydrolysis is obtained by chemical route or, alternatively, by enzymatic route.

It is known that gelatin hydrolysis obtained by chemical route, is generally carried out by heating with concentrated hydrochloric acid to a temperature of about 110°C for a time included between 18 and 24 hours. This operating modality appears of difficult application on a preparation scale. In addition, recent bibliographic references (Fountoulakis M., Lahm H.W., J. Chromatogr. A, (1998) 826 109-134) clearly highlight formation of decomposition products the nature of which is a function of the employed experimental conditions.

It is known that gelatin hydrolysis, obtained by enzymatic route is surely a less drastic process, but it has drawbacks as well.

A first drawback is connected with the presence of free sulfur amino acids derived from hydrolytic processes. Owing to the presence of sulfur amino acids the organoleptic properties of the gelatin are worsened, due to sulphur smell and aftertaste that cannot be accepted by all consumers and patients.

Alternatively, mixtures could be used that consist of the individual amino acids in the proportions found in collagen.

The last-mentioned solution too is not devoid of shortcomings. A first shortcoming refers to the related high costs for accomplishment of these amino acid mixtures. A high cost is hardly compatible with the features of a treatment involving administrations over long periods of time, in relatively high amounts.

In addition, the presence of potentially toxic impurities is not to be excluded, which impurities result from both extractive and synthetic processes employed for obtaining the individual amino acids constituting the mixture.

Abstract of application JP 11 266803 A (ISHIZUKA Y) relates to crude collagen obtained dy delimining of fish scale. Scale of hard fish is hydrolyzed in order to obtain soft collage which is not gelatinous. The soft collagen has high nutritive value when it is used as food additive. It acts as a source of aminoacid nutrition.

Application WO 00/10525 relates to fish gelatinous composition for use as an ingredient in tablets. Particulate composition comprising one or more physiologically active substances and a fish gelatinous protective colloid containing at least 50% by weight of fish gelatine, wherein the fish gelatine is at least partly composed of fish gelatine having a bloom strenght of above 100, and wherein the composition is obtainable by a particle forming spraying or double emulsifying method.

Scientific article "Role of collagen hydrolysate in bone and joint disease", Seminars in arthritis and rheumatism, 2000; 30(2):87-99, suggests that a collagen hydrolysate is of interest as a therapeutic agent of potential utily in the treatment of osteoarthritis and osteoporosis. High level of safety makes it attractive as an agent for long-term use in these chronic disorders.

The technical problem to be solved consists in making a source of amino acids more bioavailable for the metabolism of the human and animal organism, said amino acids being easily assimilable and coming from a gelatin that does not show the drawbacks of the known art.

In particular, the technical problem to be solved consists in making a source of amino acids more bioavailable for the metabolism of the human and animal organism, so that diseases arising from a lack of amino acids in the living organism can be treated while avoiding the drawbacks of the known art.

The solution to this problem is proposed by the Applicant that found it useful to employ a partly-hydrolysed fish gelatin.

It is a first object of the present invention to provide uses of a partly-hydrolysed fish gelatin as set out in the appended independent claims.

Other preferred embodiments of the present invention are described in the appended sub-claims. Further technical features and the advantages of the invention will be best understood from the following detailed description.

The partly-hydrolysed gelatin being the object of the uses of the present invention is conceived as an available source of amino acids easily assimilable by the organism. Preferably, the natural fish gelatin is submitted to a hydrolysis treatment. Hydrolysis can be carried out by chemical route or, alternately, by enzymatic route following methods that are known to those skilled in the art. The true hydrolysis is carried out until a partly-hydrolysed gelatin is obtained in which the macromolecules have a molecular weight not exceeding 50,000 Daltons. The degree of partial hydrolysis is obtained in such a manner that the native fish gelatin, following the hydrolysis process, loses its native original structure. The native structure is responsible for gelatin gelation. Hydrolysis causes breaking of the interchain bonds of the native gelatin and does not involve formation of free sulfurised amino acids. Treatment to which collagen (in this case fish collagen) is submitted brings to achievement of a gelatin form which is cold-soluble in water, which has lost its gel-forming feature. The product is defined as a non-gelling water-soluble gelatin. The particular chemical structure of partly-hydrolysed gelatin which is the object of the present invention allows the gelatin to be easily assimilable by the organism and, consequently allows use of lower dosages than those required if native gelatin were used. In addition, since the partly-hydrolysed gelatin being the object of the invention does not contain free sulfur amino acids, it has no disagreeable aftertaste which is typical of the last-mentioned products. Finally, presently there are not in the literature announcements of a possible allergenic power which was on the contrary practically found and mentioned in the literature with reference to native gelatin.

The partly-hydrolysed gelatin which is the object of the present invention achieves two important advantages with respect to both the fully hydrolysed gelatin and the reconstituted gelatin obtained by mixing the constituent amino acids thereof in their original proportion:
- it improves acceptability of treatment by a patient avoiding the disagreeable aftertaste due to free sulfur amino acids;
- it greatly reduces the treatment cost.

As compared with native gelatin, the partly-hydrolysed gelatin on the contrary has the following advantages:
- it improves treatment acceptability by the patient avoiding the feeling of gastric swelling due to gelation;
- it reduces the required doses for obtaining the desired effects.

This result could be expected in the light of the prior art that led to think that a full hydrolysis of the native gelatin was necessary and essential for improving absorption of the gelatin-constituent amino acids and reduce active dosages thereof.

The partly-hydrolysed gelatin being the object of the uses of the present invention applies to all gelatin uses that are not linked to its original physico-chemical properties, such as swelling in the presence of water, but that are linked to its ability to supply the necessary amino acids in a readily absorbable form where there is a lack of amino acids or under unhealthy conditions that can take advantage of an additional supply of amino acids.

The partly-hydrolysed fish gelatin is provided for use as a medical speciality.

The compositions comprising partly-hydrolysed gelatin efficaciously apply in the medical field and in the veterinary field. In the veterinary field, the compositions comprising partly-hydrolysed gelatin can be used with reference to pets such as dogs and cats for example.

The partly-hydrolysed fish gelatin is used for preparation of food additives for preventive and/or curative treatment of diseases associated with a lack of amino acids in the living organism. In particular, for the preventive treatment of, and/or as an adjuvant in osteoporosis, alopecia, convalescence, senescence, pregnancy, nursing, altered trophism of micro circulation and veins and in some parodontitis.

Preferably, the composition of the food additive and/or medical speciality is in a form adapted for oral administration.

In addition, the compositions for use in the present invention can be employed as adjuvants in therapies where use of other specific drugs is provided.

Advantageously, the composition of the food additive and/or medical speciality is in the form of a ready-dissolution granular powder, masticable tablets or effervescent tablets. In addition, water-based sterile solutions comprising the compositions of the present invention already in a dissolved form ready for use can be prepared.

The composition comprising the partly-hydrolysed fish gelatin being the object of the present invention is preferably in a preferably anhydrous solid form comprising mineral salts, vitamins, amino acids, flavours, pH controls, co-formulation aids, additives and substances necessary to a correct formulation of the food additive and/or medical speciality.

Preferably, vitamins are selected from the group comprising: vitamin A, vitamin B₁, vitamin B₂, vitamin B₃, vitamin B₆, vitamin C, vitamin D₃, vitamin E and vitamin H.

The composition of the uses of the present invention contains the partly-hydrolysed fish gelatin and may be in a solid form, in the form of a granular powder or micro capsules for example.

Advantageously, the partly-hydrolysed fish gelatin is in combination with calcium carbonate and vitamin D₃ (cholecalciferol).

Advantageously, the composition of the food additive and/or medical speciality efficiently applies under unhealthy conditions, such as senescence, convalescence, pregnancy, and chronic renal insufficiency and in sports activities.

In particular, the compositions of the uses object of the invention are employed in parodontitis; in particular in tooth diseases connected with both thinning of the bony tissue and weakening of dental ligaments.

These compositions efficiently apply for the above mentioned cases as an alternative to other amino acid sources, such as meat that can be assimilated less easily or can involve taking of useless or dangerous components.

In addition, they can be employed in association with low-calory diets in treating obesity in order to avoid the risk of an insufficient amino acid supply.

These compositions can be administered to patients through true treatment cycles.

For instance, a treatment cycle involves administration of an amount of partly-hydrolysed fish gelatin included from 4 to 10 g/day. Preferably, in an amount of 5-6 g/day.

For instance, administration takes place twice a day at the main meals for a period of time of 8 to 16 weeks. Preferably for a period of time in the range of 10 to 12 weeks. In addition, provision is made for a method involving preventive and/or curative treatment of diseases sharing a deficient supply or an increased consumption of amino acids, or a defect in the amino acid absorption in the organism comprising at least one treatment cycle during which a composition comprising partly-hydrolysed fish gelatin is administered.

### Oral absorption of the amino acids contained in the gelatin

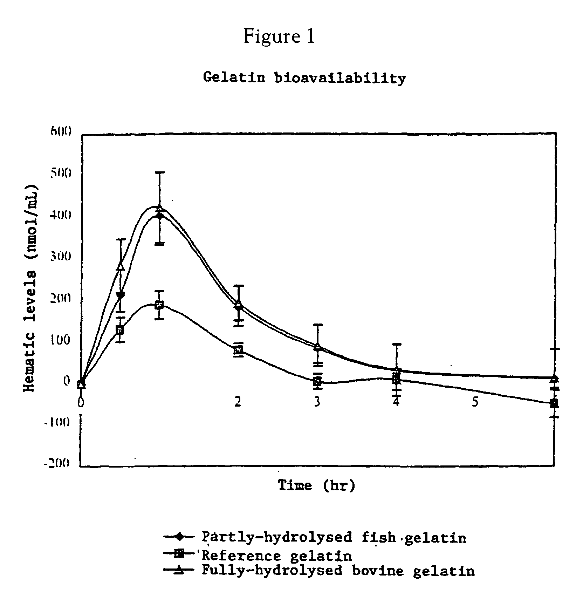

To two parallel groups of eight rabbits on an empty stomach 100 mg/kg of partly-hydrolysed (non gelling) fish gelatin with an average molecular weight not exceeding 50,000 Dalton and respectively 100 mg/kg of reference gelatin of bovine origin, non hydrolysed and with marked gelling properties at room temperature were administered through a gastric tube. At predetermined times samples of blood from the central artery of the ear were drawn to determine the plasma concentration of the free amino acids. The analysis was conducted after precipitation of the plasma proteins, lyophilizing of the residue, transformation into a derivative by phenyl isothiocyanate and HPLC analysis Pico Tag®. The quantitative determination was carried out on six amino acids present in greater quantities in the gelatin and more particularly: glycine, alanine, arginine, proline, hydroxyproline and glutamic acid. Data reproduced in Table 1 and Figure 1 are expressed in nmoles/ml as the sum of the six amino acids analysed after subtraction of basal values. From data in Table 1 and in the graph in Fig. 1, it is possible to infer that absorption of partly-hydrolysed fish gelatin, evaluated in terms of free amino acids present in the blood, is about twice with respect to the reference gelatin. The increase is almost superimposable on that previously reproduced with the fully hydrolysed gelatin. The partly-hydrolysed gelatin can be used as a base for preparations adapted for treatment of different pathologic conditions.

In addition the partly-hydrolysed gelatin can be administered in formulations of different form such as ready-dissolution granular powder, masticable pills, tablets soluble in mouth, effervescent tablets, masticable tablets and kit. By way of non-limiting example of the object of the present invention, some examples of compositions comprising partly-hydrolysed fish gelatin are reproduced hereinafter. The compositions of the following examples were prepared following knowledge and techniques known to a person skilled in the art.

| Example 1: ready-dissolution granular powder to be used in osteoporosis (1 packet of granular powder contains) : | | |
|---|---|---|
| Partly-hydrolysed fish gelatin | g | 2.50 |
| Calcium carbonate (with addition of maltodextrin) | g | 1.10 |
| Vitamin D₃ (cholecalciferol) | µg | 2.50 |
| Sodium benzosulfimide | mg | 50 |
| Strawberry/vanilla flavour | mg | 114 |
| Citric acid | g | 1.76 |
| Mannitol | mg | 250 |
| 50% dimethicone powder | mg | 60 |
| Red colour | mg | 2 |

| Example 2: tablet | | |
|---|---|---|
| Partly-hydrolysed fish gelatin | g | 2.50 |
| Calcium carbonate (with addition of maltodextrin) | g | 1.10 |
| Vitamin D₃ (cholecalciferol) | µg | 2.50 |
| Sodium benzosulfimide | mg | 50 |
| Strawberry/vanilla flavour | mg | 117 |
| Citric acid | g | 1.50 |
| Mannitol | mg | 250 |
| 50% dimethicone powder | mg | 60 |
| PEG 6000 | mg | 60 |
| Red colour | mg | 2 |

| Example 3: ready-dissolution granular powder to be used in convalescence (1 packet of granular powder contains) : | | |
|---|---|---|
| Partly-hydrolysed fish gelatin | g | 2.50 |
| Lysine hydrochloride | mg | 150 |
| Taurine | mg | 50 |
| Vitamin B₆ | mg | 1 |
| Vitamin C | mg | 45 |
| Sodium benzosulfimide | mg | 50 |
| Sodium cyclamate | mg | 50 |
| Banana flavour | mg | 154 |

| Example 4: ready-dissolution granular powder to be used in senescence (1 packet of granular powder contains): | | |
|---|---|---|
| Partly-hydrolysed fish gelatin | g | 2.50 |
| EPA- and DHA-rich sea fish oil | g | 2 |
| Vitamin E | mg | 10 |
| Vitamin C | mg | 45 |
| Betacarotene | mg | 5 |
| Sodium benzosulfimide | mg | 50 |
| Sodium cyclamate | mg | 50 |
| Vanilla flavour | mg | 150 |

| Example 5: ready-dissolution granular powder to be used in pregnancy and/or nursing (1 packet of granular powder contains): | | |
|---|---|---|
| Partly-hydrolysed fish gelatin | g | 2.50 |
| Lysine hydrochloride | mg | 150 |
| Iron gluconate (corresponding to mg 7 iron) | mg | 60 |
| Calcium glycerophosphate | g | 1 |
| Vitamin A | mg | 0.4 |
| Vitamin B₁ | mg | 0.5 |
| Vitamin B₂ | mg | 0.5 |
| Vitamin B₃ | mg | 7.5 |
| Vitamin B₆ | mg | 1 |
| Vitamin C | mg | 25 |
| Vitamin D₃ (cholecalciferol) | µg | 2.5 |
| Vitamin H | mg | 0.07 |
| Sodium benzosulfimide | mg | 50 |
| Vanilla flavour | mg | 150 |

| Example 6: ready-dissolution granular powder to be used in micro circulation and vein troubles (1 packet of granular powder contains): | | |
|---|---|---|
| Partly-hydrolysed fish gelatin | g | 2.50 |
| *Centella Asiatica* (triterpene fraction) | mg | 15 |
| Vitamin C | mg | 45 |
| Vitamin E | mg | 7 |
| Sodium benzosulfimide | mg | 50 |
| Sodium cyclamate | mg | 50 |
| Strawberry flavour | mg | 140 |

| Example 7: ready-dissolution granular powder to be used in association with low-calory diets (1 packet of granular powder contains): | | |
|---|---|---|
| Partly-hydrolysed fish gelatin | g | 2.50 |
| Sodium benzosulfimide | mg | 50 |
| Strawberry flavour | mg | 140 |

| Example 8: ready-dissolution granular powder to be used as medical speciality in osteoporosis (1 packet of granular powder contains): | | |
|---|---|---|
| Partly-hydrolysed fish gelatin | g | 2.50 |
| Calcium carbonate (with addition of maltodextrin) | g | 1.25 |
| Vitamin D₃ (cholecalciferol) | µg | 4.4 |
| Sodium benzosulfimide | mg | 50 |
| Strawberry/vanilla flavour | mg | 114 |
| Citric acid | g | 2.175 |
| Mannitol | mg | 250 |
| 50% dimethicone powder | mg | 60 |
| Red colour | mg | 2 |

**Table 1**

| Partly-hydrolysed fish gelatin | | | Reference gelatin | | | Fully-hydrolysed bovine gelatin | | |
|---|---|---|---|---|---|---|---|---|
| Time (hr) | nmol/mL | Standard error | Time (hr) | nmol/mL | Standard error | Time (hr) | nmol/mL | Standard error |
| 0 | 0 | 0 | 0 | 0 | 0 | 0 | 0 | 0 |
| 0.5 | 216.01 | 41.74 | 0.5 | 131.50 | 28.43 | 0.5 | 285.10 | 64.22 |
| 1 | 405.75 | 70.44 | 1 | 190.13 | 34.39 | 1 | 424.74 | 84.16 |
| 2 | 183.13 | 45.51 | 2 | 81.38 | 16.69 | 2 | 193.21 | 41.89 |
| 3 | 85.88 | 36.21 | 3 | 5.25 | 17.74 | 3 | 91.40 | 49.58 |
| 4 | 31.50 | 30.45 | 4 | 10.50 | 25.29 | 4 | 33.84 | 62.81 |
| 6 | 10.63 | 22.86 | 6 | -49.75 | 32.85 | 6 | 11.98 | 70.18 |

## Claims

1. Use of a partly hydrolysed fish gelatin for the preparation of a medicament for preventive and/or curative treatment of tooth diseases connected with both thinning of the bone tissue and weakening of dental ligaments.

2. Use as claimed in claim 1, wherein at least one substance selected from the group comprising: vitamin A, vitamin B₁, vitamin B₂, vitamin B₃, vitamin B₆, vitamin C, vitamin D₃, vitamin E, vitamin H, is included in said medicament.

3. Use as claimed in claim 2, wherein at least one substance selected from the group comprising: amino acids, mineral salts, flavours, pH controls, co-formulation aids and additives, is further included in said medicament.

4. Use as claimed in one or more of claims 1-3, wherein said medicament is in a form adapted for oral administration.

5. Use as claimed in one or more of claims 1-4, wherein said medicament is in the form of a granular powder, masticable tablets and effervescent tablets.

6. Use as claimed in one or more of claims 1-5, wherein the treated subjects are human beings and animals:

7. Use of a partly hydrolysed fish gelatin for the preparation of a medicament for preventive and/or curative treatment of parodontitis.

8. Use as claimed in claim 7, wherein at least one substance selected from the group comprising: vitamin A, vitamin B₁, vitamin B₂, vitamin B₃, vitamin B₆, vitamin C, vitamin D₃, vitamin E, vitamin H, is included in said medicament.

9. Use as claimed in claim 8, wherein at least one substance selected from the group comprising: amino acids, mineral salts, flavours, pH controls, co-formulation aids and additives, is further included in said medicament.

10. Use as claimed in one or more of claims 7-9, wherein said medicament is in a form adapted for oral administration.

11. Use as claimed in one or more of claims 7-10, wherein said medicament is in the form of a granular powder, masticable tablets and effervescent tablets.

12. Use as claimed in one or more of claims 7-11, wherein the treated subjects are human beings and animals.

13. Use of a partly hydrolysed fish gelatin for the preparation of a medicament for treatment of trophism of the micro circulation and veins.

14. Use as claimed in claim 13, wherein at least one substance selected from the group comprising: vitamin A, vitamin B₁, vitamin B₂, vitamin B₃, vitamin B₆, vitamin C, vitamin D₃, vitamin E, vitamin H, is included in said medicament.

15. Use as claimed in claim 14, wherein at least one substance selected from the group comprising: amino acids, mineral salts, flavours, pH controls, co-formulation aids and additives, is further included in said medicament.

16. Use as claimed in one or more of claims 13-15, wherein said medicament is in a form adapted for oral administration.

17. Use as claimed in one or more of claims 13-16, wherein said medicament is in the form of a granular powder, masticable tablets and effervescent tablets.

18. Use as claimed in one or more of claims 13-17, wherein the treated subjects are human beings and animals.

## Patentansprüche

1. Verwendung von teilweise hydrolisertem Fischgelatin zur Zubereitung eines Arzneimittels für die vorbeugende und/oder pflegende Behandlung von sowohl auf die Verminderung des Knochengewebes als auch auf Schwächung der Zahnverankerungen zurück zuführenden Zahnleiden.

2. Verwendung nach Anspruch 1, in der im Arzneimittel mindestens ein Stoff inbegriffen ist, ausgewählt aus der Gruppe bestehend in: Vitamine A, Vitamine B₁, Vitamine B₂, Vitamine B₃, Vitamine B₆, Vitamine C, Vitamine D₃, Vitamine E, Vitamine H.

3. Verwendung nach Anspruch 1, bei der im Arzneimittel mindestens ein Stoff weiter inbegriffen ist, ausgewählt aus der Gruppe bestehend in: Aminosäuren, Mineralsalze, Aromen, pH-Stabilisatoren, Mitbildner und Zusätze.

4. Verwendung nach einem oder mehreren Ansprüchen 1 bis 3, bei der das Arzneimittel sich in einer für die orale Verabreichung geeigneten Form befindet.

5. Verwendung nach einem oder mehreren Ansprüchen 1 bis 4, bei der das Arzneimittel in Form von Granulat, Kautabletten und Brausetabletten ist.

6. Verwendung nach einem oder mehreren Ansprüchen 1 bis 5, bei der die behandelten Subjekte menschliche Lebewesen und Tiere sind.

7. Verwendung von teilweise hydrolisiertem Fischgelatin zur Zubereitung eines Arzneimittels zur vorbeugenden und/oder pflegenden Behandlung von Paradentitis.

8. Verwendung nach Anspruch 7, bei der im Arzneimittel mindestens ein Stoff inbegriffen ist, gewählt aus der Gruppe bestehend in: Vitamine A, Vitamine B₁, Vitamine B₂, Vitamine B₃, Vitamine B₆, Vitamine C, Vitamine D₃, Vitamine E, Vitamine H.

9. Verwendung nach Anspruch 8, bei der im Arzneimittel mindestens ein Stoff weiter inbegriffen ist, ausgewählt aus der Gruppe bestehend in: Aminosäuren, Mineralsalze, Aromen, pH-Stabilisatoren, Mitbildner und Zusätze.

10. Verwendung nach einem oder mehreren Ansprüchen 7 - 9, bei der das Arzneimittel in einer für die orale Verabreichung geeigneten Form ist.

11. Verwendung nach einem oder mehreren Ansprüchen von 7 bis 10, bei der das Arzneimittel in Form von Granulat, Kautabletten und Brausetabletten ist.

12. Verwendung nach einem oder mehreren Ansprüchen 7 bis 11, bei der die behandelten Subjekte menschliche Lebewesen und Tiere sind.

13. Verwendung von teilweise hydrolisiertem Fischgelatin zur Zubereitung eines Arzneimittels zur Ernährungsbehandlung des Mikrokreislaufes und der Venen.

14. Verwendung nach Anspruch 13, bei der im Arzneimittel mindestens ein Stoff inbegriffen ist, gewählt aus der Gruppe bestehend in: Vitamine A, Vitamine B₁, Vitamine B₂, Vitamine B₃, Vitamine B₆, Vitamine C, Vitamine D₃, Vitamine E, Vitamine H.

15. Verwendung nach Anspruch 14, bei der im Arzneimittel mindestens ein Stoff weiter inbegriffen ist, ausgewählt aus der Gruppe bestehend in: Aminosäuren, Mineralsalze, Aromen, pH-Stabilisatoren, Mitbildner und Zusätze.

16. Verwendung nach einem oder mehreren Ansprüchen von 13 bis 15, bei der das Arzneimittel in einer zur oralen Verabreichung geeigneten Form ist.

17. Verwendung nach einem oder mehreren Ansprüchen von 13 bis 16, bei der das Arzneimittel in der Form von Granulat, Kautabletten und Brausetabletten ist.

18. Verwendung nach einem oder mehreren Ansprüchen 13 bis 17, bei der die behandelten Subjekte menschliche Lebewesen und Tiere sind.

## Revendications

1. Utilisation d'une gélatine de poisson partiellement hydrolysée pour la préparation d'un médicament pour le traitement préventif et/ou curatif des maladies des dents liées soit à une raréfaction du tissu osseux soit à un affaiblissement des ligaments des dents.

2. Utilisation selon la revendication 1, dans laquelle au moins une substance choisie parmi le groupe consistant en: vitamine A, vitamine B₁, vitamine B₂, vitamine B₃, vitamine B₆, vitamine C, vitamine D₃, vitamine E, vitamine H, est comprise dans ledit médicament.

3. Utilisation selon la revendication 2, dans laquelle au moins une substance choisie parmi le groupe consistant en: aminoacides, sels minéraux, aromates, stabilisateurs de pH, aides de co-formulation et additifs est également comprise dans ledit médicament.

4. Utilisation selon une ou plusieurs revendications 1-3, dans laquelle ledit médicament est dans une forme apte à l'administration orale.

5. Utilisation selon une ou plusieurs revendications 1-4, dans laquelle ledit médicament est dans la forme d'une poudre granulaire, de comprimés qu'on peut mâcher, et de comprimés effervescents.

6. Utilisation selon une ou plusieurs revendications 1-5, dans laquelle les sujets traités sont des êtres humains et des animaux.

7. Utilisation d'une gélatine de poisson partiellement hydrolysée pour la préparation d'un médicament pour le traitement préventif et/ou curatif de périodontite.

8. Utilisation selon la revendication 7, dans laquelle au moins une substance choisie parmi le groupe consistant en: vitamine A, vitamine B₁, vitamine B₂, vitamine B₃, vitamine B₆, vitamine C, vitamine D₃, vitamine E, vitamine H, est comprise dans ledit médicament.

9. Utilisation selon la revendication 8, dans laquelle au moins une substance choisie parmi le groupe consistant en: aminoacides, sels minéraux, aromates, stabilisateurs de pH, aides de co-formulation et additifs est également comprise dans ledit médicament.

10. Utilisation selon une ou plusieurs revendications 7-9, dans laquelle ledit médicament est dans une forme apte à l'administration orale.

11. Utilisation selon une ou plusieurs revendications 7-10, dans laquelle ledit médicament est dans la forme d'une poudre granulaire, de comprimés qu'on peut mâcher, et de comprimés effervescents.

12. Utilisation selon une ou plusieurs revendications 7-11, dans laquelle les sujets traités sont des êtres humains et des animaux.

13. Utilisation d'une gélatine de poisson partiellement hydrolysée pour la préparation d'un médicament pour le traitement de la trophicité de la microcirculation et des veines.

14. Utilisation selon la revendication 13, dans laquelle au moins une substance choisie parmi le groupe consistant en: vitamine A, vitamine B₁, vitamine B₂, vitamine B₃ vitamine B₆, vitamine C, vitamine D₃, vitamine E, vitamine H, est comprise dans ledit médicament.

15. Utilisation selon la revendication 14, dans laquelle au moins une substance choisie parmi le groupe consistant en: aminoacides, sels minéraux, aromates, stabilisateurs de pH, aides de co-formulation et additifs est également comprise dans ledit médicament.

16. Utilisation selon une ou plusieurs revendications 13-15, dans laquelle ledit médicament est dans une forme apte à l'administration orale.

17. Utilisation selon une ou plusieurs revendications 13-16, dans laquelle ledit médicament est dans la forme d'une poudre granulaire, de comprimés qu'on peut mâcher, et de comprimés effervescents.

18. Utilisation selon une ou plusieurs revendications 13-17, dans laquelle les sujets traités sont des êtres humains et des animaux.
